# EUROPEAN PATENT SPECIFICATION

(11) **EP 3 311 840 B1**
(45) Date of publication and mention of the grant of the patent: **29.09.2021**
(21) Application number: 16811690.3
(22) Date of filing: 16.06.2016
(51) Int. Cl.: A61K 39/395, A61K 45/06, A61K 41/00, A61P 35/00

(54) **TREATMENT-RESISTANCE REDUCING AGENT FOR TREATMENT-RESISTANT CANCER**
MITTEL ZUR VERRINGERUNG DER BEHANDLUNGSRESISTENZ FÜR BEHANDLUNGSRESISTENTEN KREBS
AGENT DE RÉDUCTION DE RÉSISTANCE À UN TRAITEMENT POUR CANCER RÉSISTANT À UN TRAITEMENT

(30) Priority: 17.06.2015 JP 2015122399
(43) Date of publication of application: 25.04.2018
(73) Proprietor: NATIONAL UNIVERSITY CORPORATION HOKKAIDO UNIVERSITY, Kita-ku Sapporo-shi Hokkaido 060-0808 (JP)
(72) Inventor: BAGHDADI, Muhammad, Sapporo-shi Hokkaido 0600808 (JP); SEINO Ken-ichiro, Sapporo-shi, Hokkaido 0600808 (JP)
(74) Representative: Wasner, Marita
(86) International application number: PCT/JP2016/067906
(87) International publication number: WO 2016/204216

(56) References cited:
- WO-A1-2016/097420
- WO-A1-2016/168149
- JP-A- 2013 529 183
- JP-A- 2014 520 873
- JP-A- 2015 510 510
- US-A1- 2011 274 683
- Y.A. LUQMANI: "Mechanisms of Drug Resistance in Cancer Chemotherapy", MEDICAL PRINCIPLES AND PRACTICE, vol. 14, no. 1, 1 January 2005 (2005-01-01), pages 35-48, XP055541145, CH ISSN: 1011-7571, DOI: 10.1159/000086183
- BAGHDADI, M. ET AL.: 'IL -34 accelerates the formation of immunosuppressive macrophages in chemoresistant tumors' PROCEEDINGS OF THE JAPANESE SOCIETY FOR IMMUNOLOGY vol. 44, October 2015, page 111, XP009507886
- COPISETTY, A. ET AL.: 'Prosurvival and chemoprotective effects of tumor-associated macrophages reversed by anti-CSF-1R monoclonal antibody in B- cell lymphomas' BLOOD vol. 124, no. 21, 2014, page 498, XP009507888
- 'CSF1R ANTIBODY OVERVIEW' LILLY ONCOLOGY, [Online] 06 July 2016, XP009507931 Retrieved from the Internet: <URL:HTTP://LILLYONCOLOGYPIPELINE.COM/MOLEC ULE/CSF-1-R-ANTIBODY/OVERVIEW>
- DENARDO, D.G. ET AL.: 'Leukocyte complexity predicts breast cancer survival and functionally regulates response to chemotherapy' CANCER DISCOVERY vol. 1, no. 1, 2011, ISSN 2159-8274 pages OF52 - OF65, XP055106141
- WEIZMAN, N. ET AL.: 'Macrophages mediate gemcitabine resistance of pancreatic adenocarcinoma by upregulating cytidine deaminase' ONCOGENE vol. 33, 2014, ISSN 0950-9232 pages 3812 - 3819, XP055337633
- ESCAMILLA, J. ET AL.: 'CSF1 receptor tergeting in prostate cancer reverses macrophage-mediated resistance to androgen blockade therapy' CANCER RESEARCH vol. 75, no. 6, March 2015, ISSN 0099-7013 pages 950 - 962, XP055337636
- PAULUS, P. ET AL.: 'Colony-Stimulating factor-1 antibody reverses chemoresistance in human MCF- 7 breast cancer xenografts' CANCER RESEARCH vol. 66, no. 8, 2006, ISSN 0099-7013 pages 4349 - 4356, XP055149182
- PALMA, M.D. ET AL.: 'Macrophage regulation of tumor responses to anticancer therapies' CANCER CELL vol. 23, 2013, ISSN 1535-6108 pages 277 - 286, XP055337641
- JINUSHI, M. ET AL.: 'Tumor-associated macrophages as an emerging target against tumors: Creating a new path from bench to bedside' BIOCHIMICA ET BIOPHYSICA ACTA vol. 1855, January 2015, ISSN 0304-419X pages 123 - 130, XP029575043

### SEQUENCE LISTING

<110> Seino, Ken-ichiro
<120> Therapy-resistance reducing agent for therapy-resistant cancer
<130> P15014WO
<160> 12
<170> PatentIn version 3.5
<210> 1
   <211> 24
   <212> DNA
   <213> Artificial Sequence
<220>
   <223> IL-34 Forward Primer
<400> 1
   ctttgggaaa cgagaatttg gaga 24
<210> 2
   <211> 24
   <212> DNA
   <213> Artificial Sequence
<220>
   <223> IL-34 Reverse Primer
<400> 2
   gcaatcctgt agttgatggg gaag 24
<210> 3
   <211> 20
   <212> DNA
   <213> Artificial Sequence
<220>
   <223> M-CSF Forward Primer
<400> 3
   ctgaagagct gcttcaccaa 20
<210> 4
   <211> 20
   <212> DNA
   <213> Artificial Sequence
<220>
   <223> M-CSF Reverse Primer
<400> 4
   atggtggtgt ccttgacaac 20
<210> 5
   <211> 20
   <212> DNA
   <213> Artificial Sequence
<220>
   <223> CD14 Forward Primer
<400> 5
   gccgctgtgt aggaaagaag 20
<210> 6
   <211> 20
   <212> DNA
   <213> Artificial Sequence
<220>
   <223> CD14 Reverse Primer
<400> 6
   aggttcggag aagttgcaga 20
<210> 7
   <211> 21
   <212> DNA
   <213> Artificial Sequence
<220>
   <223> CD11b Forward Primer
<400> 7
   ctggaaaggg agacttttca c 21
<210> 8
   <211> 20
   <212> DNA
   <213> Artificial Sequence
<220>
   <223> CD11b Reverse Primer
<400> 8
   aagatgccca ctgaggaatg 20
<210> 9
   <211> 22
   <212> DNA
   <213> Artificial Sequence
<220>
   <223> CD68 Forward Primer
<400> 9
   gctacatggc ggtggagtac aa 22
<210> 10
   <211> 22
   <212> DNA
   <213> Artificial Sequence
<220>
   <223> CD68 Reverse Primer
<400> 10
   atgatgagag gcagcaagat gg 22
<210> 11
   <211> 20
   <212> DNA
   <213> Artificial Sequence
<220>
   <223> CD163 Forward Primer
<400> 11
   tcgagttaac gccagtaagg 20
<210> 12
   <211> 20
   <212> DNA
   <213> Artificial Sequence
<220>
   <223> CD163 Reverse Primer
<400> 12
   tcagaacatg tcacgccagc 20

## Description

### Technical Field

The present invention relates to a treatment-resistance reducing agent for a treatment-resistant cancer, containing, as an active ingredient(s), a substance inhibiting the binding between interleukin-34 (IL-34) and colony stimulating factor-1 receptor (CSF-1R).

### Background Art

Therapeutic treatments for cancers include, for example, a surgical treatment in which cancer tissues are removed, chemotherapy in which anticancer agents are administered, radiation therapy in which cancer tissues are irradiated, and cancer immunotherapy in which immunity against cancer is induced. Among these, treatments other than the surgical treatment have the problem that the implementation of such a treatment turns the cancer into a treatment-resistant cancer showing resistance to the treatment.

In particular, the emergence of treatment-resistant cancers in chemotherapy and radiation therapy is a serious problem. In chemotherapy and radiation therapy, owing to their nature, it is inherently difficult to treat cancer tissues in strict distinction from normal tissues, and there are problems, for example, that chemotherapy is prone to cause side effects because of small differences between efficacy and toxicity, and that radiation therapy inevitably necessitates irradiation to normal tissues around the cancer tissue. For these reasons, once the cancer has acquired resistance to the treatment, it will be necessary to administer a larger amount of the anticancer agent or irradiate the cancer tissue at a larger dose or in a higher intensity. The cancer treatment must often be discontinued when the side effects may become intolerable or the exposure dose of the normal tissue may be too high.

In particular, administration of an anticancer agent, chemotherapy, which is the most common therapeutic treatment, simultaneously leads to the generation of many treatment-resistant cancers, i.e., drug-resistant cancers. Examples of anticancer agents causing practical problems of drug-resistant cancers include, for example, doxorubicin, tamoxifen, cisplatin, and docetaxel, which are used for the treatment of breast cancer and ovarian cancer; and Iressa and Tarceva, which are therapeutic drugs for non-small cell lung cancer.

The identification of the mechanism by which cancer cells acquire or exert treatment resistance, and of molecules involved in this mechanism, and in addition, the development of techniques preventing the involvement of these molecules are useful for solving the problem of treatment resistance of cancers and also for enhancing the effectiveness of the existing therapeutic treatments.

Attention is also focused on the development of a new method for cancer treatment that can be expected to be effective even for treatment-resistant cancers. In particular, the inhibition of the immunosuppressive action caused by cancer cells is a promising therapeutic target; for example, an inhibitor of an immune checkpoint molecule which has the function of inhibiting the activity of host immune cells is under vigorous development as a new anticancer agent that cancels the immunosuppression caused by cancer cells and activates host immune responses.

CSF-1R (Colony Stimulating Factor-1 Receptor, also referred to as M-CSFR, CD-115, or c-fms) is a single chain transmembrane receptor tyrosine kinase having an immunoglobulin (Ig) motif in the extracellular domain, which is registered as Uniprot Accession No. P07333.

M-CSF (macrophage colony-stimulating factor, also referred to as CSF-1) and IL-34 (Non-Patent Literature 1) are known as specific ligands of CSF-1R. IL-34 is a human cytokine, which is registered as Uniprot Accession No. Q6ZMJ4, and the base sequence of the gene encoding it is registered as Gene ID: 146433 in the NCBI (National Center for Biotechnology Information).

It has been confirmed that, through the tyrosine kinase activity of CSF-1R, M-CSF and IL-34 are associated with cell differentiation, proliferation and migration, and with the survival of precursor macrophages derived from the monocyte lineage and of osteoclasts, while it is thought that these two ligands are different such as in the ability to induce the production of chemokines including MCP-1 and eotaxin 2 in primary macrophages, the morphological change in TF-1-fms cells, the migration of J774A.1 cells (Non-Patent Literature 2).

It has been reported that as medicaments targeting IL-34 and/or CSF-1R, isolated antibodies capable of inhibiting the binding between CSF-1R and IL-34 by means of binding to IL-34 are used for the treatment of rheumatoid arthritis, inflammatory bowel disease, multiple sclerosis, macrophage activation syndrome (MAS), discoid lupus erythematosus, sarcoidosis, vasculitis, and myelogenous immune diseases such as graft versus host disease (Patent Literature 1). It has also been reported that antibodies capable of binding to CSF-1R and inhibiting the binding between CSF-1R and IL-34 are used for the treatment of leukemia, breast cancer, endometrial cancer, prostate cancer, ovarian cancer, colorectal cancer, hepatocellular carcinoma, kidney cancer, multiple myeloma, and others (Patent Literature 2). Patent Literature 3 relates to methods of treating cancer with antibodies that bind colony stimulating factor 1 receptor (CSF-1R) in combination with one or more immune stimulating agents. Moreover, it has been reported that an antibody against CSF-1R overcomes treatment resistance of cancer (Non-Patent Literature 3).

However, the relationship between IL-34 and treatment resistance of cancers has not been observed yet.

### Citation List

### Patent Literatures

Patent Literature 1: JP 2015-510510 W
Patent Literature 2: JP 2013-529183 W
Patent Literature 3: WO2016/168149

### Non-Patent Literatures

Non-Patent Literature 1: Lin et al., Science, 2008, vol. 320, 807-811.
Non-Patent Literature 2: Chihara et al., Cell Death and Differentiation, 2010, vol. 17, 1917-1927.
Non-Patent Literature 3: Copisetty et al., Blood, vol 124, no. 21, 2014, 498

### Summary of Invention

### Technical Problem

The present invention aims to provide a treatment-resistance reducing agent for a treatment-resistant cancer which can eliminate or reduce the treatment resistance, particularly drug resistance, of the cancer and enhance the effectiveness of a cancer treatment.

### Solution to Problem

The present inventors found experimentally that IL-34 was involved in drug resistance in the course of studying the mechanism relating to drug resistance, which is a type of resistance to cancer treatments, and have completed the inventions described below.

(1) A substance specifically inhibiting the binding between IL-34 and
   CSF-1R by means of binding to IL-34 for use in reducing treatment resistance of a cancer, wherein the substance is an antibody specific to IL-34.
(2) The substance for the use according to claim 1, for use in combination with a chemotherapy, radiation therapy and/or immunotherapy.

### Advantageous Effects of Invention

According to the present invention, the treatment resistance of cancer, which is resistant to a treatment method such as the administration of an anticancer agent or radiation therapy, can be reduced and, therefore, the efficacy of cancer treatment can be enhanced and the occurrence risk of a side effect can be suppressed.

### Brief Description of Drawings

FIG. 1 shows results of FACS analysis after stimulation of peripheral blood lymphocytes (PBL) derived from a healthy human donor with culture supernatants of doxorubicin (DOX)-sensitive A549 human lung adenocarcinoma cell lines (A549-DS) or DOX-resistant A549 cell line (A549-DR). The upper diagrams in FIG. 1 represent dot plots for CD14 and CD11b on the cells after the stimulation, and the lower graph in FIG. 1 shows the percentage of CD14⁺CD11b⁺ cells (cells in the bold-lined box of the respective diagrams).
FIG. 2 represents microscopic images after stimulation of human healthy donor-derived monocytes with GM-CSF, M-CSF, or culture supernatants of A549-DS or A549-DR cells.
FIG. 3 represents graphs showing mRNA expression levels of CD14, CD11b, CD68, and CD163 after stimulation of human healthy donor-derived monocytes with GM-CSF, M-CSF, or culture supernatants of A549-DS or A549-DR cells. The horizontal axis of the graphs indicates the relative values compared to the respective expression levels in the control (monocytes cultured in DMEM alone).
FIG. 4 represents a graph showing the expression amount of M-CSF mRNA in A549-DR cells as a relative value to that in A549-DS cells.
FIG. 5 represents a graph showing the concentration of IL-34 in culture supernatants of A549-DS or A549-DR cells.
FIG. 6 represents graphs showing mRNA expression levels of CD14, CD11b, CD68, and CD163 after stimulation of human healthy donor-derived monocytes with an A549-DR cell culture supernatant, an A549-DR cell culture supernatant after treatment with an anti-IL-34 neutralizing antibody, or an A549-DR-IL-34-KO cell culture supernatant. The vertical axis of the graphs indicates the relative values compared to the respective expression levels in the control (monocytes cultured in DMEM alone).
FIG. 7 represents graphs showing the cell viability for A549-DS, A549-DR, and A549-DR-IL-34-KO cells (the upper graph in FIG. 7) and for A549-DR cells neutralized with an anti-IL-34 antibody (the lower graph in FIG. 7), cultured in the presence or absence of DOX.
FIG. 8 represents graphs showing the change in tumor size by administration of DOX or PBS in humanized mice that had been inoculated with A549-DS, A549-DR, or A549-DR-IL-34-KO cells. The horizontal axis of the left graph in FIG. 8 represents the number of days after the inoculation of cancer cells.
FIG. 9 represents a graph showing the number of CD68⁺CD163⁺ cells in tumors from humanized mice that had been inoculated with A549-DS, A549-DR, or A549-DR-IL-34-KO cells.

### Description of Embodiments

Through the experiments shown below in 1) to 5), the present inventors found that IL-34 expressed in cancer cell lines was involved in treatment resistance of cancers.

### 1) Generation of drug-resistant cancer cells

Human lung adenocarcinoma cell line A549 was obtained from the American Type Cell Culture (ATCC, USA). Cell culture was carried out at 37°C in an atmosphere of 5% CO2 using Dulbecco's Modified Eagle's Medium (DMEM) (SIGMA) supplemented with fetal bovine serum (at a final concentration of 10%), penicillin (at a final concentration of 100 U/mL) and streptomycin (at a final concentration of 100 µg/mL). A549 cells were cultured at 37°C in an atmosphere of 5% CO₂, in DMEM in which the DOX concentration was increased stepwise from 0.01 to 1 µM, and then cells acquiring DOX resistance were selected to generate a DOX-resistant A549 cell line (A549-DR). The resultant A549-DR cells maintained their drug resistance by exposure to 1 µM DOX.

### 2) Effects of culture supernatants of A549-DR cells on the differentiation of monocytes

A549-DR cells generated above in 1) were washed 5 times with sterilized PBS and seeded to be at a density of 1 × 10⁶ cells/mL in a cell culture plate in which 10 mL of DMEM without DOX was dispensed in advance, and cultured for 72 hours, followed by collecting the culture supernatant. A culture supernatant of A549 cells which had not been subjected to the induction of drug resistance (A549-DS) was also prepared in a similar way.

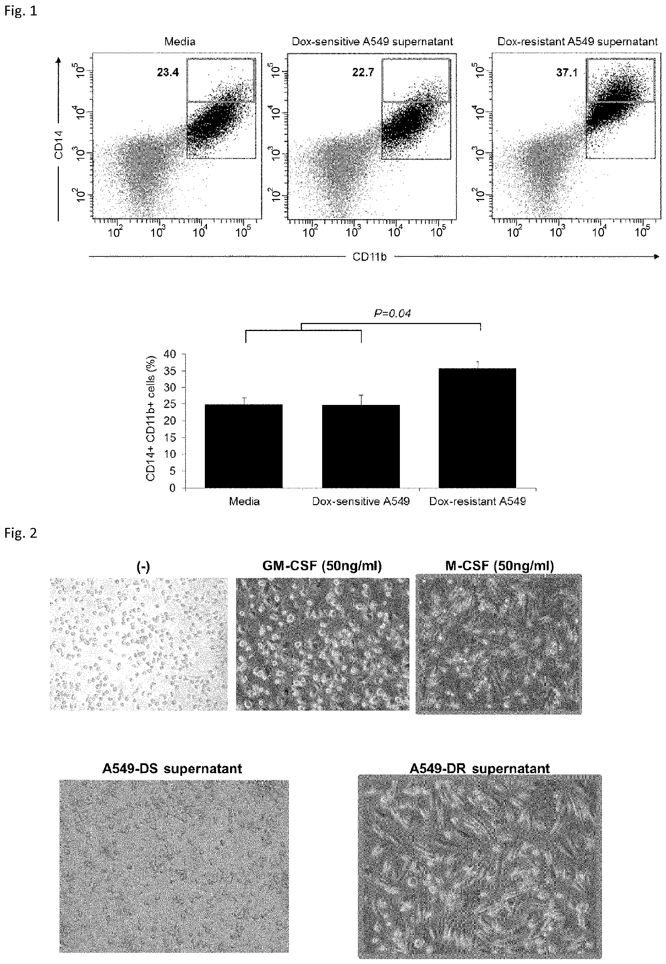

Peripheral blood lymphocytes (PBL) were prepared from peripheral blood of a human healthy donor according to routine procedures, and stimulated by culturing them in DMEM or in DMEM to which the A549-DS or A549-DR cell culture supernatant was added to 50%. After one week of culturing, the floating cells were collected and subjected to FACS analysis for CD14 and CD11b, which are cell surface antigens expressed in monocytes and macrophages, using a flow cytometer (Biolegend, FACS Canto II) (monoclonal antibody reagents: Human Anti CD14 and Human Anti CD11b, both available from Biolegend). The results are shown in Fig. 1.

In the case of stimulation with the A549-DR cell culture supernatant, an increase in the number of CD14⁺CD11b⁺ cells was observed, as compared with the case of stimulation with the medium or with the A549-DS cell culture supernatant. This result suggested that a factor(s) affecting the differentiation of monocytes was present in the A549-DR cell supernatant.

### 3) Investigation on a monocyte differentiation inducing factor(s) contained in the A549-DR cell culture supernatant

CD14⁺ monocytes were isolated from a human healthy donor using a magnetic cell sorting system (Miltenyi Biotech), and stimulated with culture supernatants of A549-DS or A549-DR cells in a similar manner as in 2) above. After 6 to 7 days, monocytes were collected, and total RNA was extracted with Sepazol (nacalai) to determine expression levels of CD14, CD11b, CD68 and CD163, which are known as macrophage markers, by RT-PCR. RT-PCR was performed using the Power SYBR (registered trademark) Green (Applied Biosystems) with the primer sets described in Table 1, according to the manufacturer's protocol.

**Table. 1**

| | Forward Primer | Reverse Primer |
|---|---|---|
| IL-34 | 5'-CTTTGGGAAACGAGAATTTGGAGA-3' (SEQ ID NO. 1) | 5'-GCAATCCTGTAGTTGATGGGGAAG-3' (SEQ ID NO. 2) |
| M-CSF | 5'-CTGAAGAGCTGCTTCACCAA-3' (SEQ ID NO. 3) | 5'-ATGGTGGTGTCCTTGACAAC-3' (SEQ ID NO. 4) |
| CD14 | 5'-GCCGCTGTGTAGGAAAGAAG-3' (SEQ ID NO. 5) | 5'-AGGTTCGGAGAAGTTGCAGA-3' (SEQ ID NO. 6) |
| CD11b | 5'-CTGGAAAGGGAGACTTTTCAC-3' (SEQ ID NO. 7) | 5'-AAGATGCCCACTGAGGAATG-3' (SEQ ID NO. 8) |
| CD68 | 5'-GCTACATGGCGGTGGAGTACAA-3' (SEQ ID NO. 9) | 5'-ATGATGAGAGGCAGCAAGATGG-3' (SEQ ID NO. 10) |
| CD163 | 5'-TCGAGTTAACGCCAGTAAGG-3' (SEQ ID NO. 11) | 5'-TCAGAACATGTCACOCCAGC-3' (SEQ ID NO. 12) |

For comparison, monocytes were stimulated with GM-CSF (25 ng/mL) or M-CSF (25 ng/mL) for their differentiation, and then subjected to an evaluation in a similar way. It is known that monocytes differentiate into M1 macrophages when stimulated with GM-CSF and into M2 macrophages when stimulated with M-CSF.

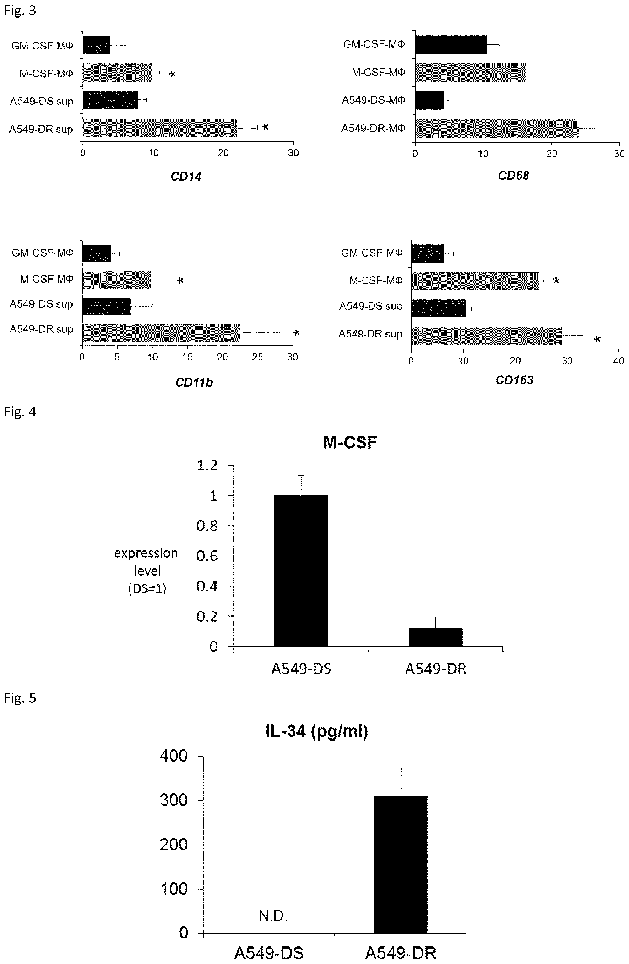

FIG. 2 shows results of the morphological observation of cells after the stimulation, and FIG. 3 shows the comparison of the expression levels of CD14, CD11b, CD68 and CD163. It was found that in the presence of the A549-DR cell culture supernatant, monocytes differentiated into macrophages, of which the morphology was similar to that of the macrophages induced by M-CSF.

### 4) Analysis of the expression of M-CSF and IL-34 in A549-DR cells

On the basis of the results described above in 3), it was hypothesized that A549-DR cells secrete M-CSF, allowing monocytes to differentiate into M2 macrophages. To address this hypothesis, the expression of M-CSF mRNA in A549-DR cells was determined by RT-PCR. RT-PCR was carried out using the primer sets described in Table 1, in a similar manner as in 3) above. The results are shown in Fig. 4. Contrary to the hypothesis, the expression level of M-CSF mRNA was lower in A549-DR cells than in A549-DS cells.

Since the macrophages induced by the A549-DR cell culture supernatant had similar morphology and phenotype to those of the M-CSF induced macrophages, there could be the possibility that A549-DR cells produce another ligand of CSF-1R which is a receptor for M-CSF, and thus attention was paid to IL-34, known as a ligand of CSF-1R. FIG. 5 shows the results of measurement of IL-34 in culture supernatants of A549-DS and A549-DR cells using an ELISA kit (Biolegend). No IL-34 was detected in the A549-DS culture supernatant, while a large amount of IL-34 was found in the A549-DR cell culture supernatant.

### 5) Effect of IL-34 deletion on the monocyte differentiation ability of A549-DR cell culture supernatant

In order to elucidate the role of IL-34 in drug resistance, an IL-34 knockout A549-DR cell line (A549-DR-IL-34-KO) was generated using the CRISPR system. Specifically, IL34-gene knockout kit via CRISPR, Human (-) (Origene) having a guide RNA for IL-34 incorporated in pCas-guide vector was used for transfection into A549-DR cells using FuGENE (registered trademark) 6 Transfection Reagent (Promega) as a transfection reagent. The knockout of the IL-34 gene was confirmed by the absence of detection of IL-34 mRNA and protein by RT-PCR and ELISA.

A culture supernatant of resultant A549-DR-IL-34-KO cells was prepared in a similar manner as in 2) above to evaluate the effect on the differentiation of monocytes. In addition, an IL-34 neutralization treatment was performed by adding an anti-IL-34 neutralizing antibody (R & D systems, MAB 5265, 10 µg/mL) to an A549-DR cell culture supernatant obtained in 2) above, followed by reaction for 60 minutes, and after that, the neutralized supernatant was evaluated in a similar way.

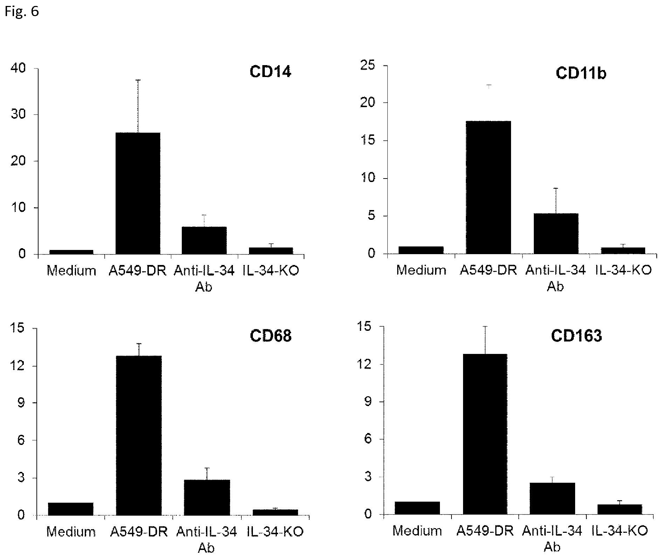

FIG. 6 shows the comparison of the expression levels of CD14, CD11b, CD68 and CD163. The IL-34 neutralization or IL-34 knockout lead to a reduced increase in the expression level of the cell surface antigens observed on cells that had been stimulated with the A549-DR cell culture supernatant. Accordingly, it was ascertained that by the deletion of IL-34, the A549-DR cell culture supernatant lost the ability to differentiate monocytes into M2 macrophages.

From the results described above and the Examples described later, it was shown that A549-DR cells, which are drug-resistant cancer cells, maintained their drug resistance through the secretion of produced IL-34 and in addition thereto, promoted the differentiation of monocytes into immunosuppressive M2 macrophages. M2 macrophages present within or in the vicinity of tumor tissues are also known as Tumor Associated Macrophages (TAMs), and it has been reported that the accumulation of TAMs in a tumor tissue is in part responsible for impairing the effectiveness of chemotherapy or radiation therapy (Tang et al., Immunology, 2012, 138, 93-104). It is also known that IL-34 promotes the differentiation of monocytes into immunosuppressive M2 macrophages by means of binding to CSF-1R as a ligand thereof. Therefore, it is expected that the suppression of the expression of IL-34 in a cancer cell or inhibition of the binding between IL-34 and CSF-1R makes it possible to prevent cancers from acquiring treatment resistance through IL-34.

A treatment-resistance reducing agent of the present invention contains, as an active ingredient(s), a substance specifically inhibiting the binding between IL-34 and human CSF-1R. A treatment-resistance reducing agent of the present invention can be applied both to a resistant cancer that is resistant to a treatment by nature and to an acquired resistant cancer that has acquired resistance to a treatment due to exposure thereto, and the application to the latter acquired resistant cancer is preferable.

The substance specifically inhibiting the binding between IL-34 and human CSF-1R is preferably a substance which, as an active ingredient, inhibits the binding between IL-34 and human CSF-1R by means of binding to CSF-1R or IL-34, particularly by means of specific binding to IL-34. Example of such a substance can include, for example, an anti-IL-34 antibody that inhibits the binding between IL-34 and CSF-1R through blocking the CSF-1R binding site on IL-34; or an anti-IL-34 antibody that binds to IL-34 at a site different from said binding site on IL-34, and inhibits, with steric hindrance, the binding between IL-34 and CSF-1R. Since CSF-1R binds to M-CSF besides IL-34, it is preferable in the present invention to use IL-34-binding antibodies, in that they do not affect the binding between M-CSF and CSF-1R.

The antibody used in the present invention can be a monoclonal antibody, a chimeric antibody, a humanized antibody or a human antibody. Antibody fragments, such as Fab, Fab', or F(ab')2, of these antibodies can also be used in the present invention.

These antibodies can be prepared by common methods for antibody production including immunizing a suitable laboratory animal, such as a rabbit, a mouse, and a rat, using preferably recombinant human IL-34 produced by genetic recombination as an antigen. Alternatively, existing anti-IL-34 antibodies, such as those described in the above-mentioned Patent Literature 1, MAB 5265, which is an anti-IL-34 antibody commercially available from R & D systems and others, can be used.

A treatment-resistance reducing agent of the present invention containing an above-described antibody as an active ingredient is preferably prepared and used in the form of lyophilized preparations or aqueous solutions using common carriers used for antibody-containing pharmaceutical preparations. Examples of such carriers can include, but are not limited to, buffers, antioxidants, preservatives, proteins, hydrophilic polymers, amino acids, chelating agents, nonionic surfactants, and others.

The treatment-resistance reducing agent of the present invention can be used in the form of pharmaceutical compositions formed or formulated with pharmaceutically acceptable excipients, carriers, medicaments and the like other than those exemplified above. Pharmaceutically acceptable ingredients are well known to those skilled in the art; a person skilled in the art is able to select and use them as appropriate, depending on the form of formulations, within the scope of ordinary capacities for implementation, for example, from the ingredients described in the Japanese Pharmacopoeia, Sixteen Edition, and other standards.

The treatment-resistance reducing agent or a pharmaceutical composition containing the same of the present invention is preferably administered parenterally, such as by intramuscular administration, intravenous administration, intraarterial administration, intraperitoneal administration, subcutaneous administration, or by direct administration to a cancer tissue, and preferably is in the form of preparations suitable for these administration routes, such as injections, drops, or others. The dosage amount is selected as appropriate, depending on the usage, the age of the patient, the type of the disease, other conditions, and the like.

The treatment-resistance reducing agent or the pharmaceutical composition containing the same in the present invention is preferably used in combination with chemotherapy in which an anticancer agent is administered, radiation therapy and/or immunotherapy. In particular, preference is given to combined use with chemotherapy in which an anticancer agent is administered. Examples of anticancer agents effective for use in combination with a treatment-resistance reducing agent or a pharmaceutical composition containing the same of the present invention can include, but are not limited to, for example, doxorubicin, tamoxifen, cisplatin, and docetaxel.

It is expected that by administration to a patient having a treatment resistant cancer, the treatment-resistance reducing agent of the present invention can reduce the treatment resistance of the cancer, thereby enhance the effectiveness of cancer treatments by chemotherapy, radiation therapy and/or immunotherapy. Accordingly, it can be said that the present invention provides a substance specifically inhibiting the binding between IL-34 and CSF-1R by means of binding to IL-34 for use in reducing treatment resistance of a cancer, including administering an effective amount of a pharmaceutical composition to a patient with a resistant cancer, wherein the pharmaceutical composition contains a substance specifically inhibiting the binding between IL-34 and human CSF-1R, wherein the substance is an antibody specific to IL-34. Furthermore, it can also be said that the present invention provides a substance specifically inhibiting the binding between IL-34 and CSF-1R by means of binding to IL-34 for use in reducing treatment resistance of a cancer or preventing the metastasis of a cancer, including administering an effective amount of a pharmaceutical composition to a patient with a resistant cancer, wherein the pharmaceutical composition contains a substance specifically inhibiting the binding between IL-34 and human CSF-1R, wherein the substance is an antibody specific to IL-34; and wherein the patient receives a chemotherapy, radiation therapy and/or immunotherapy, together with or separately from, simultaneously with or sequentially with the administering.

The present invention will be further described in detail with reference to the following Examples.

### Examples

### Example 1. In vitro confirmation of the effect of reducing DOX resistance

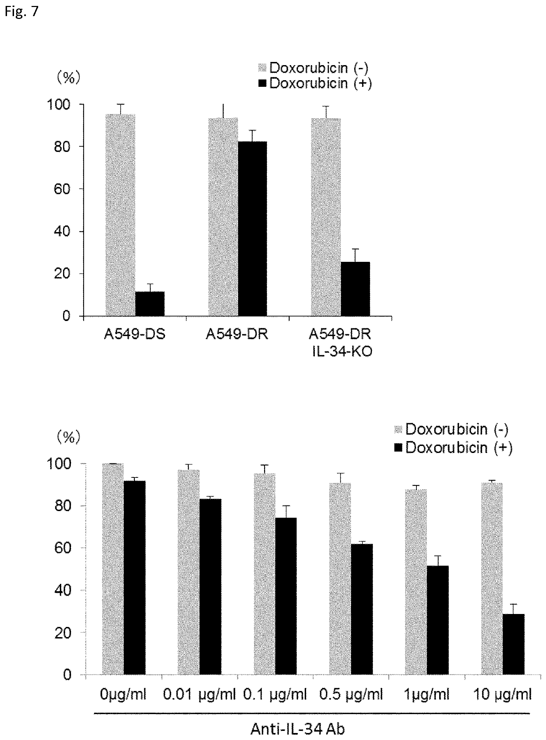

A549-DS, A549-DR, and A549-DR-IL-34-KO cells were cultured in DMEM medium with or without DOX (at a final concentration of 1 µM). After 72 hours of culturing, an MTT assay was performed (using a cell proliferation determination kit, available from Roche Applied) to determine the viability of cells. Similarly, A549-DR cells were cultured in a medium with or without DOX in which an anti-IL-34 neutralizing antibody was added at a final concentration of 0.01 to 10 µg/mL, and the viability of cells was determined in a similar way. The results are shown in Fig. 7.

A549-DR cells did not have a decreased viability and exhibited DOX resistance even in the presence of DOX. On the other hand, A549-DR-IL-34-KO cells and A549-DR cells neutralized with the anti-IL-34 antibody restored the sensitivity to DOX, confirming the reduction of DOX resistance by the deletion of IL-34.

### Example 2. In vivo confirmation of the effect of reducing DOX resistance

NOG mice (NOD/Shi-Scid IL-2 Rγ KO Jic, female, 6 weeks old) were irradiated with X-ray (2.5 G), and 24 hours later, transplanted with two millions of human bone marrow cells (Stem cell technologies). After 3 weeks, the mice were inoculated with 1 × 10⁶ A549-DS, A549-DR, or A549-DR-IL-34-KO cells. When the tumor size reached 5 mm (i.e., 1 week after the inoculation of cancer cells), DOX was intravenously administered at a dose of 10 mg/kg/mouse, twice a week, 4 times in total. In the control group, PBS was administered in the same amount, instead of DOX. After the completion of the administration test, the tumor tissue was removed from each of the mice to prepare a single cell suspension, which was then stained with anti-CD68 and anti-CD163 (manufactured by Biolegend), followed by evaluating the number of CD68⁺CD163⁺ cells by FACS analysis.

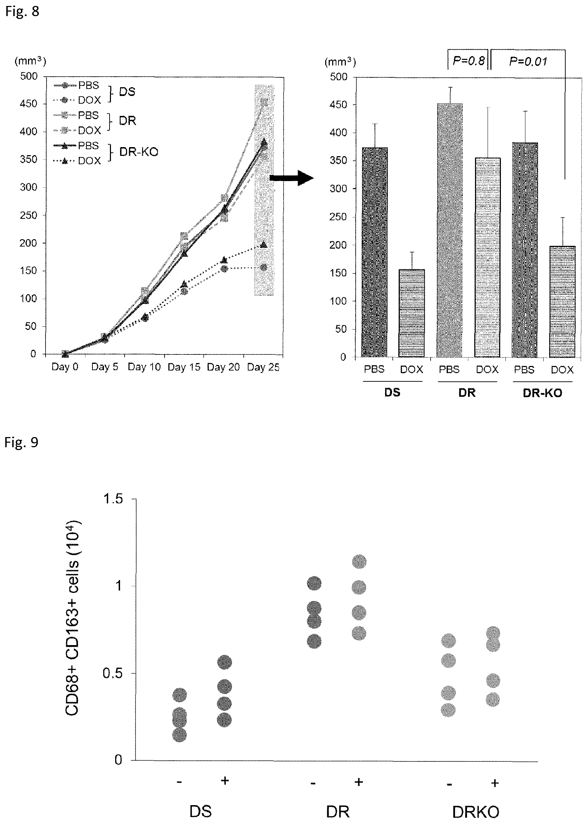

The changes in tumor size over time are shown on the left in FIG. 8, and the tumor sizes at the end of the test are shown on the right in FIG. 8. The size of the A549-DR tumor did not change greatly with DOX administration, whereas the size of the A549-DR-IL-34-KO tumor was significantly decreased with DOX administration.

FIG. 9 shows the number of CD68⁺CD163⁺ cells, i.e., M2 macrophages, in the respective tumors. The number of M2 macrophages infiltrating into the A549-DR tumor was greater than that in the A549-DS tumor. On the other hand, the A549-DR tumor in which IL-34 had been knocked out had a decreased number of M2 macrophages.

### Industrial Applicability

The treatment-resistance reducing agent of the present invention for treatment-resistant cancer can be used in combination with chemotherapy, radiation therapy or immunotherapy, for treating cancer, whereby it has industrial applicability as a medicament capable of maintaining the effectiveness of those therapies.

## Claims

1. A substance specifically inhibiting the binding between IL-34 and CSF-1R by means of binding to IL-34 for use in reducing treatment resistance of a cancer, wherein the substance is an antibody specific to IL-34.

2. The substance for the use according to claim 1, for use in combination with a chemotherapy, radiation therapy and/or immunotherapy.

## Patentansprüche

1. Substanz, die spezifisch die Bindung zwischen IL-34 und CSF-1R durch Bindung an IL-34 inhibiert, zur Verwendung bei der Verringerung der Behandlungsresistenz von Krebs, wobei die Substanz ein für IL-34 spezifischer Antikörper ist.

2. Substanz zur Verwendung nach Anspruch 1, zur Verwendung in Kombination mit einer Chemotherapie, Strahlentherapie und/oder Immuntherapie.

## Revendications

1. Substance inhibant spécifiquement la liaison entre IL-34 et CSF-1 R par liaison à l'IL-34 pour une utilisation dans la réduction de la résistance au traitement d'un cancer, la substance étant un anticorps spécifique de l'IL-34

2. Substance pour l'utilisation selon la revendication 1, pour une utilisation en combinaison avec une chimiothérapie, une radiothérapie et/ou une immunothérapie.
